# FASCICULE DE BREVET EUROPEEN

(11) **EP 1 974 720 B1**
(45) Date de publication et mention de la délivrance du brevet: **13.02.2013**
(21) Numéro de dépôt: 08153220.2
(22) Date de dépôt: 25.03.2008
(51) Int. Cl.: A61K 8/99, A61K 35/74, A61Q 15/00, C12P 1/04, A61P 31/04, A61P 17/00, A61P 17/02, A61P 17/10, A61P 17/08, A61Q 11/00, A61Q 19/00, A61Q 5/00, A61Q 17/00, A61K 8/60, A61Q 3/00

(54) **Utilisation d'une fraction lipopolysaccharidique de vitreoscilla filiformis comme agent stimulant la synthese de peptides antimicrobiens de la peau**
Verwendung einer Lipopolysaccharidfraktion von Vitreoscilla filiformis als Stimulationssubstanz für die Synthese von antimikrobiellen Peptiden in der Haut
Use of a vitreoscilla filiformis lipopolysaccharide fraction as an agent for stimulating the synthesis of anti-microbial skin peptides

(30) Priorité: 26.03.2007 FR 0754039
(43) Date de publication de la demande: 01.10.2008
(73) Titulaire: L'Oréal, 75008 Paris (FR)
(72) Inventeur: Mahe, Yann, 91700, Sainte Geneviève des bois (FR); Martin, Richard, 37210 Rochecorbon (FR)
(74) Mandataire: Allab, Myriam

(56) Documents cités:
- EP-A- 0 876 813
- EP-A- 1 354 593
- EP-A- 1 531 158
- FR-A- 2 693 654
- FR-A- 2 879 452
- FR-A- 2 879 461
- US-A- 6 036 966
- US-B1- 6 645 506
- HASEGAWA N ET AL: "ELEVATED PROMOTION OF PROSTACYCLIN PRODUCTION BY SYNTHETIC LIPID A ANALOGS IN AGED HUMAN ENDOTHELIAL CELLS IN CULTURE" MECHANISMS OF AGEING AND DEVELOPMENT, ELSEVIER SEQUOIA, LAUSANNE,, CH, vol. 78, no. 2, 1995, pages 155-162, XP001181663 ISSN: 0047-6374
- HARDER JüRGEN ET AL: "Antimicrobial peptides in human skin" CHEMICAL IMMUNOLOGY AND ALLERGY, KARGER, BASEL, CH, vol. 86, 2005, pages 22-41, XP009088004 ISSN: 1660-2242

## Description

La présente invention se rapporte à l'utilisation du Lipide A de *Vitreoscilla filiformis,* ou d'une fraction spécifique de *Vitreoscilla filiformis* comprenant son Lipide A, pour stimuler la synthèse de peptides antimicrobiens de la peau.

La peau humaine est constituée de deux compartiments à savoir un compartiment superficiel, l'épiderme et un compartiment profond, le derme.
L'épiderme est composé principalement de trois types de cellules qui sont les kératinocytes (majoritaires), les mélanocytes et les cellules de Langerhans qui jouent un rôle primordial dans la réponse immunitaire et en particulier dans la présentation antigénique.
Le derme fournit à l'épiderme un support solide. C'est également son élément nourricier. Il est principalement constitué de fibroblastes et d'une matrice extracellulaire. On y trouve aussi des leucocytes, des mastocytes et des macrophages tissulaires. Enfin, Le derme est traversé par des vaisseaux sanguins et des fibres nerveuses.
La peau constitue une barrière contre les agressions extérieures, notamment chimiques, mécaniques, et à ce titre un certain nombre de réactions de défense contre les facteurs environnementaux (climat, rayons ultraviolets, tabac, pollutions...) et/ou les xénobiotiques (comme par exemple certains médicaments) se produisent à son niveau. De plus, la peau est le siège de réactions allergiques telles qu'observées dans les cas d'eczémas de contact ou d'eczémas atopiques.

On sait que les cellules épidermiques sont capables de répondre à des agressions extérieures microbiennes (bactériennes ou fongiques) en produisant des peptides et des protéines à activité anti-microbienne.
Ces peptides et protéases de défense (dont les Béta défensines, la RNAse 7, la Psoriasine, voir l'article « Antimicrobial peptides in human skin » de Schröder et al. Mechanisms of epithelial défense, Chem Immunol Allergy, 2005, vol 86 pp 22-41) sont normalement produites par les cellules de l'épiderme après contact avec des agents pathogènes intacts et vivants.
Dans des conditions normales, cette production de peptides suffit à limiter la prolifération de microorganismes sur la peau évitant ainsi qu'elle ne soit lésée ou infectée.

Cependant, il arrive que ces systèmes ne suffisent pas à limiter la prolifération bactérienne et on assiste alors à des infections ou sepsies de la peau et/ou des muqueuses épithéliales.

On comprend alors tout l'intérêt qu'il y a de pouvoir stimuler ce premier système de défense naturelle afin de rétablir une homéostasie naturelle, notamment dans des situations ou la fonction barrière est déficiente que ce soit partiellement (peaux sèches, atopie...) ou totalement (brûlures, plaies, dermabrasion, traitement de peeling).
On cherche ainsi à mimer l'activité des bactéries entières sur la peau avec un composé appliqué directement sur la peau, l'utilisation d'un composé mimétique à la place d'un microorganisme vivant permet d'éviter tout risque de prolifération bactérienne indésirable.

On sait par ailleurs que les Lipo Poly Saccharides (LPS) des membranes externes d'*Escherichia coli* sont capables de mimer certains des effets de la bactérie entière pour stimuler la réponse des cellules immunitaires via notamment la production de cytokines dites pro inflammatoires (comme l'interleukine 1, le TNF alpha) ou encore les C-C et CXC chemokines comme L'IL-8 et le MCP-1 pour recruter et activer certaines populations de cellules lymphocytaires.
Paradoxalement, ces LPS d'*E*. *coli,* s'ils sont actifs par voie systémique ou par voie intraveineuse sur les cellules immunitaires, sont très faiblement actifs, voire inactifs, pour activer directement la première barrière de défense cutanée (i.e. les kératinocytes). Ainsi, ils apparaissent inefficaces pour induire les systèmes de défense cutanés superficiels contre les microorganismes.

La Demanderesse a maintenant mis en évidence qu'une fraction spécifique de LPS comprenant le Lipide A de la bactérie filamenteuse non fructifiante, non photosynthétique, *Vitreoscilla filiformis* a la capacité de stimuler l'expression de peptides anti-microbiens de la peau par les kératinocytes. Contrairement à *E. coli,* cette bactérie ne présente aucune activité pathogène.

Cette fraction LPS spécifique a ensuite été évaluée in vitro par rapport à une fraction équivalente d'un LPS d' *E. coli* (C14-C16) préparée dans les mêmes conditions et à la même concentration finale (10µg/ml) pour son éventuelle propriété à induire spécifiquement la synthèse des précurseurs (ARNms) de peptides antibactériens épidermiques.
Il a ainsi été observé, de façon surprenante et inattendue, que comparée au LPS d' *E. coli,* la fraction spécifique de Lipide A de *Vitreoscilla filiformis,* aux mêmes concentrations et dans les mêmes conditions expérimentales, était capable de stimuler plus efficacement la mise en route préventive des systèmes de défense cutanés, notamment anti-bactériens.
Ainsi, cette fraction a pour propriété biologique de se comporter comme un « leurre » qui active les systèmes de défense cutanée sans en produire les inconvénients liés à l'activité intrinsèque d'un agent pathogène ou d'une bactérie complète.

Divers extraits de *Vitreoscilla filiformis* dotés de propriétés immunostimulantes non spécifiques ont été décrits par la Demanderesse en vue d'une utilisation cosmétique ou pharmaceutique, en particulier dans le traitement des signes cutanés du vieillissement. C'est le cas des demandes EP 0 604 631 et EP 0 765 667 qui utilisent, respectivement, des extraits de bactéries filamenteuses non fructifiantes, et une fraction de ces bactéries riche en ribosomes obtenue par centrifugation de la biomasse et dialyse du surnageant obtenu.
La demande EP 0 876 813 décrit une fraction immunostimulante obtenue à partir du milieu de culture de ces bactéries. La demande WO 94/02158 divulgue l'utilisation d'enveloppes de bactéries ou de fractions, notamment de LPS, obtenues à partir desdites enveloppes comme agent stimulant du système immunitaire.
La demande EP 1 531 158 décrit l'utilisation du lipide A pour renforcer les défences immunitaires cutanées.
Enfin, la demande EP 1 400 237 décrit un activateur pouvant être un extrait total de *Vitreoscilla filiformis* pour améliorer la résistance de la peau aux effets non spécifiques de l'activation du complément.

La présente invention se rapporte à l'utilisation de Lipide A de la bactérie filamenteuse non fructifiante non photosynthétique *Vitreoscilla filiformis* en tant qu'agent inducteur de l'expression de peptides anti-microbiens par la peau. Le Lipide A est un constituant de la fraction lipopolysacharidique (LPS).

La souche bactérienne *Vitreoscilla filiformis* (souche déposée à l'ATCC 15551) est utilisée actuellement par la Demanderesse pour la fabrication d'une biomasse introduite dans des produits cosmétiques. Le procédé de préparation de cette biomasse comprend la culture en continu des bactéries en milieu stérile oxygéné, en présence de sels minéraux et de sucres ; le prélèvement, puis la centrifugation, du milieu de culture comprenant les bactéries, pour obtenir une biomasse qui est mise en flacons puis stérilisée. L'avantage de travailler en continu, et donc à taux de croissance µ constant, est la garantie d'un état physiologique constant lui aussi. L'éclatement des cellules résultant de la stérilisation provoque une décantation de la biomasse en une boue renfermant essentiellement des membranes cellulaires et des protéines coagulées et contenant environ 70% du poids de LPS, et en un surnageant comprenant le cytoplasme et contenant environ 30% du poids de LPS, la bactérie entière contenant (en poids sec) environ 10% de LPS. Une biomasse homogène est reconstituée par agitation avant emploi.

Classiquement, le procédé de préparation d'une fraction LPS peut être réalisé selon des techniques publiées.
A titre d'exemple, on peut citer la technique de référence dite technique de Westphal et coll. (phenol/chloroform/petrol ether) de 1952 ou bien la technique de Galanos et coll., 1969, (phenol : eau) ou encore la technique dérivée de Westphal : Westphal & Jann [Methods Carbohydr. Chem. 5 (1965) 83], la technique de Galanos et al. [Eur. J. Biochem. 9 (1969) 245], celle de Ni Eidhin et Mouton [FEMS Microbiol. Lett. 110 (1993) 133] et celle de Nichols [Infect. Immun. 62(1994) 3753].
Toutes ces techniques sont applicables pour extraire un LPS.
Il est cependant important de préciser que ces techniques ne permettent pas d'obtenir des fractions très purifiées de LPS contrairement à la technique indiquée dans ce qui suit.

Pour préparer une fraction spécifique comprenant le Lipide A, la méthode de Caroff M. suivant la demande de brevet WO2004/062690 intitulée « Novel method for isolating endotoxins » peut être mise en oeuvre. Cette méthode utilise un mélange d'acide isobutyrique et d'ammoniaque en solution aqueuse molaire dans un rapport volumique de 5/3 ou un mélange d'acide isobutyrique et de triéthylamine en solution aqueuse à 10% dans un rapport volumique de 5/3, ensuite le mélange est agité 10 minutes à température ambiante puis filtré et/ou centrifugé à 3000 g durant 15 minutes à 4°C. Le solvant résiduel est ensuite éliminé par évaporation sous vide.

Le Lipide A a été caractérisé après avoir été isolé à partir d'une souche de *Vitreoscilla filiformis* (EP 1 531 158).

Le lipide A des bactéries Gram - est un dimère de glucosamine porteur par condensation, sur ses groupes hydroxyles situés en positions 3 et 3', ainsi que sur ses groupes amino, situés en positions 2 et 2', d'acides gras plus ou moins insaturés et hydroxylés qui peuvent eux-mêmes êtres estérifiés, sur leurs groupes hydroxyles, par d'autres acides gras. Ce sont ces acides gras qui permettent l'ancrage du lipide A dans la membrane externe de la cellule, qui est de nature phospholipidique. En outre, leur nature (en C₁₂-C₁₈) et leur positionnement sur les glucosamines, déterminent et l'activité biologique et la toxicité pour une grande partie des lipides A et donc des LPS.

Le Lipide A selon l'invention présente la structure suivante : (a) les composés de formule (I) : 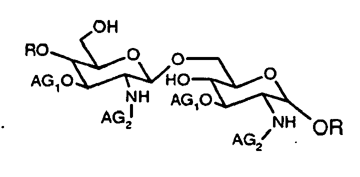 dans laquelle :
AG₁ désigne un groupe 3-hydroxy décanoyle
AG₂ désigne un groupe 3-dodécanoyloxy décanoyle,
où R désigne un atome d'hydrogène ou un groupe PO(OR')₂, R' désignant un atome d'hydrogène, un groupe alkyle linéaire ou ramifié, saturé ou insaturé, en C₁-C₆, ou un groupe phényle ou benzyle, et
(b) dans le cas où R désigne un groupe PO(OH)₂ dans la formule (I) ci-dessus, d'un sel inorganique du composé de formule (I), ou d'un sel d'amine primaire, secondaire ou tertiaire du composé de formule (I), ou d'un sel de phosphoéthanolamine du composé de formule (I),
étant entendu que, lorsque plusieurs groupements R, respectivement R', sont présents, ils sont identiques ou différents les uns des autres.

Le sel d'amine du composé de formule (I) peut être choisi parmi les sels de mono-, di- et triéthanolamine, les sels de mono-, di- ou tri-isopropanol-amine, le 2-amino 2-méthyl 1-propanol, le 2-amino 2-méthyl 1,3-propanediol et le tris(hydroxyméthyl)amino méthane.

Les sels inorganiques du composé de formule (I) peuvent notamment être des sels de sodium, magnésium, potassium, zinc ou calcium.

Par Lipide A, on entend ainsi un Lipide A tel que défini ci-avant obtenu par synthèse chimique (exemple 1), un Lipide A extrait de *Vitreoscilla filiformis* ou encore une fraction de *Vitreoscilla filiformis* enrichie en Lipide A, il pourra plus particulièrement s'agir d'une fraction LPS spécifique.
La quantité de lipide A dans la fraction LPS spécifique utilisable selon l'invention dépendra bien sûr du procédé d'extraction mis en oeuvre, les fractions LPS spécifiques enrichies en Lipide A selon l'invention comprennent au moins 0,01% en poids de Lipide A.

Par peau, on entend toute la surface du corps, incluant la peau, les muqueuses et semi-muqueuses, cuir chevelu, ainsi que leurs annexes (ongles, poils, cheveux...).

Par peptides anti-microbiens, on entend des peptides exprimés notamment par les kératinocytes lors de leur mise en contact avec des agents pathogènes microbiens, certains de ces peptides ont été décrits par Schröder *et al.* dans Antimicrobial peptides in human skin et comprennent les béta-defensines, parmi lesquelles la Human β-Defensin-1, Human β -Defensin-2, Human β-Defensin-3, Human β-Defensin-4, la Cathelicidine LL-37, la Serine Protease Inhibitor Antileukoprotease, l'elafine, la dermcidine, l'adrenomedulline, la neurophile Gelatinase-Associated lipocaline, la RNase 7. La S100 A7 (ou psoriasine) fait aussi partie de ces peptides à activité anti-microbienne capables d'interférer avec la prolifération d'agents pathogènes bactériens.

Les défensines sont des peptides antimicrobiens actifs sur les bactéries, les champignons et les virus (Harder J et coll., Nature 387 : 861, 1997 ; Frohm M et coll., J Biol Chem 272 : 15258-15263, 1997 ; Gropp R et coll., Hum Gene Ther 10 : 957-964, 1999). Ces peptides sont produits par les kératinocytes de la peau et certaines cellules des muqueuses, elles peuvent détruire la membrane des microbes cibles et/ou pénétrer leur membrane, interférant alors avec les fonctions intracellulaires des microorganismes pathogènes. La β-defensine 2 et la cathelicidine (LL-37) sont des défensines inductibles au niveau de la peau et leur augmentation est entraînée lors des inflammations ou des stress cutanés (Harder J et coll., Nature 387 : 861, 1997 ; Frohm M et coll., J Biol Chem 272 : 15258-15263, 1997). On connaît actuellement plus d'une douzaine de défensines identifiées au niveau des gènes DEFB1; DEFB2 ; DEFB124 ; DEFB107, DEF alpha6 ; DEFB106 ; DEFB122 ; DEFB105, DEFB103 ; DEFB108, DEFalpha4 ; DEFB 125 ; DEFB 124 ; DEFB 104 (appelée aussi parfois DEFB4).

L'invention se rapporte à l'utilisation du Lipide A de *Vitreoscilla filiformis* caractérisé en ce qu'il est constitué d'un composé choisi parmi :
(a) les composés de formule (I) : 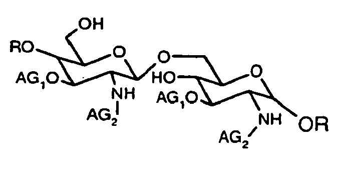 dans laquelle :
   AG₁ désigne un groupe 3-hydroxy décanoyle
   AG₂ désigne un groupe 3-dodécanoyloxy décanoyle,
   où R désigne un atome d'hydrogène ou un groupe PO(OR')₂, R' désignant un atome d'hydrogène, un groupe alkyle linéaire ou ramifié, saturé ou insaturé, en C₁-C₆, ou un groupe phényle ou benzyle, et
(b) dans le cas où R désigne un groupe PO(OH)₂ dans la formule (I) ci-dessus, d'un sel inorganique du composé de formule (I), ou d'un sel d'amine primaire, secondaire ou tertiaire du composé de formule (I), ou d'un sel de phosphoéthanolamine du composé de formule (I), pour la préparation d'une composition destinée à inhiber la prolifération d'une flore microbienne, en particulier, bactérienne cutanée indésirable, à prévenir et/ou traiter les infections et surinfections microbiennes de la peau, telle que définie par les revendications.
Ces infections ou surinfections sont des désordres cutanés notamment liés au développement de microorganismes pathogènes cutanés choisis parmi les bactéries, levures ou des champignons inférieurs (fungi) tels que, par exemple les bactéries suivantes : *Staphylococcus aureus, Streptococcus pyogenes, Pseudomonas aeruginosa, Escherichia coli, Clostridium pefringens, Clostridium difficile, Gardnerella vaginalis, Propionibacterium acnes, Klebsiella species, Streptopyogenes* ou les levures pathogènes telles que *Candida albicans, Malassezia furfur, Trichophyton mentagrophytes, Trichophyton interdigitale, Trichophyton rubrum, Trichophyton yaoundei, Tinea capitis, Tinea corporis,* ou des fungi tels que des *Aspergillus sp.*

Les désordres cutanés pourront être des complications infectieuses des désordres dermatologiques, notamment l'acné ; des complications infectieuses pendant la cicatrisation ; des dermatophytoses ; des candidoses ; des vaginoses ; des onychomycoses ; des teignes du cuir chevelu, du corps ; des désordres cutanés liés à des thérapeutiques avec des antibiotiques ou des antimycosiques ou entraînés par des dysrégulations hormonales ; des polydermites ; des érysipèles ; des dermites séborrhéiques.
En particulier, les surinfections pourront être :
- les dermatites atopiques surinfectées, l'eczéma impétiginisé, l'acné inflammatoire surinfecté, l'herpès surinfecté ;
- des surinfections des plaies et lésions cutanées quelque soit leur origine, notamment pendant la cicatrisation et sont choisies parmi les ulcères, les plaies, les brûlures ;
- les dermatophytoses telles que la teigne du cuir chevelu, la teigne du corps, le pied d'athlète, l'eczéma marginé de Hebra, l'herpès circiné ;
- les candidoses telles que les candidoses des muqueuses, les candidoses vaginales, les candidoses interdigitales, les candidoses liées à des professions à risques ou au diabète ;
- les polydermites telles que parmi l'impétigo, les folliculites superficielles.

Cette utilisation peut également être destinée à maintenir et/ou restaurer une écoflore normale La flore microbienne saprophyte ou écoflore cutanée représente un facteur majeur de protection immunitaire de la peau.
Tout déséquilibre dans la population de cette flore entraîne un déficit immunitaire fonctionnel et, bien souvent, l'occupation du territoire cutané par une flore pathogène.
La flore cutanée est estimée à 10⁶ cellules/cm² (Leyden JJ et coll., Soc Inves Dermatol 88 : 65-69, 1987), et est principalement constituée par des corynebactéries *(Corynebacterium, Brevibacterium et Propionibacterium)* et des staphylocoques.
*Acinetobacter* et des *Micrococcus* sont aussi retrouvés mais à un moindre degré, avec la flore fongique comprenant principalement le genre *Malassezia* (Noble WC, Micobiol Human Skin, London, ed Saunders Company Ltd, 1974; Leeming JP et coll., J Clin Microbiol 25 : 2017-2019, 1987; Leeming JP et coll., J Applied Bacteriol 67 : 47-52, 1989; Rudolf R et coll., J Am Acad Dermatol 1989).
Lorsque cette barrière écologique de microorganismes est défaillante (tel dans les eczémas atopiques), réduite (chez les nourrissons), voire détruite (comme après l'utilisation abusive de produits agressifs pour la peau), tout microorganisme indésirable est alors capable de proliférer sur la peau ou même de la traverser déclenchant ainsi des mécanismes de défenses non spécifiques.
La présence de l'écoflore permet donc d'assurer à la peau une ligne de défense écologique en s'opposant à l'implantation de microorganismes pathogènes par un phénomène de compétition nutritionnelle et par la sécrétion de substances aux activités enzymatiques et bactéricides.

Selon un autre aspect, la présente invention se rapporte à l'utilisation du Lipide A de *Vitreoscilla filiformis* en tant qu'agent normalisant la flore cutanée.

Par normaliser la flore cutanée, on entend maintenir ou restaurer un profil normal de population de microorganismes cutanés sur la peau, le cuir chevelu et/ou les muqueuses, c'est-à-dire une population de microorganismes correspondant à celle présente sur une peau saine, un cuir chevelu sain ou une muqueuse saine.

L'utilisation selon l'invention concerne la préparation d'une composition comprenant au moins le Lipide A de *Vitreoscilla filiformis,* ladite composition étant destinée à maintenir et/ou restaurer une écoflore cutanée normale.

En particulier, l'utilisation selon l'invention pourra être destinée :
- à prévenir et/ou limiter les états pelliculaires. A titre d'exemple, l'extrait selon l'invention pourra alors être formulé dans une composition telle qu'un shampooing, une lotion ou un tonique capillaire, un masque à laisser poser sur le cuir chevelu ;
- à la préparation de composition destinée à l'hygiène buccale. A titre d'exemple, l'extrait selon l'invention pourra alors être formulé dans une composition telle qu'une pâte dentifrice, une pâte à mâcher, une solution buccale de rinçage de la bouche ;
- à prévenir et/ou limiter les états séborrhéiques, en particulier, dans le cas de la prolifération de microorganismes pathogènes cutanés qui entretiennent le phénomène de peaux et/ou de cuir chevelu gras. A titre d'exemple, l'extrait selon l'invention pourra alors être formulé dans des crèmes de soin telles que des crèmes astringentes pour peaux grasses, des compositions nettoyantes pour le corps, le visage, les cheveux, telles que des shampooings anti-séborrhéiques ;
- à la préparation de composition destinée à l'hygiène corporelle, en particulier intime, ou capillaire. A titre d'exemple, l'extrait selon l'invention pourra alors être formulé dans des compositions nettoyantes pour la toilette intime ou pour le corps, le visage, les cheveux ou le scalp tels que des savons, gels douches, gels intimes, gels nettoyant visage, dans des produits de soin tels que des gels antibactériens après-rasage, lait pour le visage ou le corps ;
- à prévenir et/ou limiter les mauvaises odeurs liées à la prolifération de microorganismes cutanés dans les zones confinées du corps comme par exemple, la zone axillaire ou les pieds.

Pour ce type d'application, l'extrait selon l'invention pourra alors être formulé dans des compositions déodorantes.

L'utilisation selon l'invention peut être faite de telle sorte que le Lipide A est appliqué topiquement sur la peau avec ou sans poil, le cuir chevelu, les muqueuses et/ou semi-muqueuses.

Les compositions orales utilisables selon l'invention présentent préférentiellement un support ingérable.
Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, capsules. En particulier, le(s) actif(s) selon l'invention peuvent être incorporés dans toutes autres formes de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.
Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suppléments oraux sous forme sèche.

Cette application peut être réalisée sur tout mammifère, de préférence l'homme et les animaux de compagnie ou d'élevage.

Ces applications peuvent être élargies à toute composition destinée à des traitements vétérinaires. Elles pourront également être plus spécifiquement destinées à la prévention des proliférations microbiennes, en particulier bactériennes. A titre d'exemple sans caractère limitatif, il peut s'agir d'une application sur le pis des vaches et, de façon générale, de tous mammifères de production laitière.

Cette utilisation vétérinaire permettra notamment de traiter et/ou la prévenir des désordres liés à des infections staphylococciques, streptococciques et mycosiques.

Pour toutes ces utilisations, il est avantageux d'utiliser le Lipide A de *Vitreoscilla filiformis* en association avec des probiotiques et/ou des prébiotiques.

Les microorganismes probiotiques convenant à l'invention sont des microorganismes qui peuvent être administrés sans risques à l'animal ou l'homme.
Au sens de la présente invention, on entend par "microorganisme probiotique" (ou encore probiotique), un microorganisme vivant qui, lorsqu'il est consommé en quantité adéquate, a un effet positif sur la santé de son hôte "Joint FAO/WHO Expert Consultation on Evaluation of Health and Nutritional Properties of Probiotic in Food Including Powder Milk with Live Lactic Acid Bacteria, 6 octobre 2001", et qui peut en particulier améliorer l'équilibre microbien intestinal.
Selon une variante de l'invention, ce microorganisme est mis en oeuvre sous une forme isolée, c'est-à-dire non mélangée à un ou des composé(s) susceptible(s) de lui être associé(s) dans son milieu d'origine.
Les microorganismes convenant à l'invention peuvent être choisis notamment parmi les ascomycètes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus* et leurs mélanges.
Comme ascomycètes convenant tout particulièrement à la présente invention, on peut en particulier citer *Yarrowia lipolitica et Kluyveromyces lactis,* de même que *Saccharomyces cereviseae, Torulaspora, Schizosaccharamyces pombe, Candida* et *Pichia.*
En ce qui concerne les microorganismes probiotiques, ce sont les genres bactériens et de levure suivants qui sont généralement utilisés :
- les bactéries lactiques : qui produisent par fermentation du sucre de l'acide lactique. Suivant leur morphologie, on les divise en deux groupes :
   - *Lactobacillus species : Lactobacillus acidophilus ; amylovorus, casei, rhamnosus, brevis, crispatus, delbrueckii (subsp bulgaricus, lactis), fermentum, helveticus, gallinarum, gasseri johnsonii, paracasei, plantarum, reateri, salivarius, alimentarius, curvatus, casei subsp. casei, sake*
   - *Gocci : Enterococcus (faecalis, faecium), Lactococcus lactis (subspp lactis ou cremoris), Leuconstoc mesenteroides* subsp *dextranicum, Pediococcus acidilactici, Sporolactobacillus inulinus, Streptococcus salvarius subsp. Thermophilus, Streptococcus thermophilus, Staphylococccus carnosus, Staphylococcus xylosus*
- Les bifidobactéries ou *Bifidobacterium species : Bifidobacterium adolescentis, animalis, bifidum, breve, lactis, longum, infantis, pseudocatenulatum*
- Les *levures : Saccharomyces (cerevisiae* ou encore *boulardii),*
- Les autres bactéries *sporulées : Bacillus (cereus var toyo ou subtilis), Bacillus coagulans, Bacillus licheniformis, Escherichia coli strain nissle, Propionibacterium freudenreichii,*
- et leurs mélanges.

Les bactéries lactiques et les bifidobactéries sont les probiotiques le plus souvent utilisés. Des exemples spécifiques de microorganismes probiotiques sont *Bifidobacteriam adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus (NCFB 1748) ; Lactobacillus amylovorus, Lactobacillus casei (Shirota), Lactobacillus rhamnosus* (souche GG), *Lactobacillus brevis, Lactobacillus crispatus, Lactobacillus delbrueckii (subsp bulgaricus, lactis), Lactobacillus fermentum, Lactobacillus helveticus, Lactobacillus gallinarum, Lactobacillus gasseri, Lactobacillus johnsonii (CNCM I-1225), Lactobacillus paracasei, Lactobacillus plantarum, Lactobacillus reuteri, Lactobacillus salivarius, Lactobacillus alimentarius, Lactobacillus curvatus, Lactobacillus casei subsp. casei, Lactobacillus sake Lactococcus lactis, Enterococcus (faecalis, faecium), Lactococcus lactis (subspp lactis ou cremoris), Leuconstoc mesenteroides* subsp *dextranicum, Pediococcus acidilactici, Sporolactobacillus inulinus, Streptococcus salvarius subsp. Thermophilus, Streptococcus thermophilus, Staphylococccus carnosus, Staphylococcus xylosus, Saccharomyces (cerevisiae* ou encore *boulardii), Bacillus (cereus var toyo ou subtilis), Bacillus coagulans, Bacillus licheniformis, Escherichia coli strain nissle, Propionibacterium freudenreichii* et leurs mélanges.

Les microorganismes peuvent être formulés à l'état de poudres, c'est-à-dire sous une forme sèche, ou sous forme de suspensions ou de solutions.

Plus particulièrement, il s'agit de microorganismes probiotiques issus du groupe des bactéries lactiques, comme notamment les *Lactobacillus* et/ou les *Bifidobacterium.* A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges.

Les espèces convenant tout particulièrement sont les *Lactobacillus johnsonii, Lactobacillus paracasei, Bifidobacterium adolescentis, Bifidobacterium longum* et *Bifidobacterum lactis NCC 2818* respectivement déposées suivant le traité de Budapest avec l'Institut Pasteur (28 rue du Docteur Roux, F-75024 Paris cedex 15) les 30/06/92, 12/01/99, 15/04/99, 15/04/99, 07/06/05 sous les désignations suivantes CNCM I-1225, CNCM I-2116, CNCM I-2168 et CNCM I-2170 et CNCM I-3446, et le genre *Bifidobacterium longum (BB536) et leurs mélanges.*

Les microorganismes et/ou leurs fractions et/ou métabolites peuvent être formulés dans un support approprié en une quantité équivalente à au moins 10³ ufc/g, en particulier à des doses variant de 10⁵ à 10¹⁵ ufc/g, et plus particulièrement de 10⁷ à 10¹² ufc/g de support.

Les compositions à application topique ou à administration orale selon l'invention comprennent généralement de 10³ à 10¹² ufc, en particulier de 10⁵ à 10¹⁰ ufc et plus particulièrement de 10⁷ à 10⁹ ufc de microorganismes notamment probiotiques par gramme de support.

Le ou les microorganisme(s) peu(ven)t être inclus dans la composition selon l'invention sous une forme vivante, semi-active ou inactivée, morte.

Il(s) peu(ven)t également être inclus sous forme de fractions de composants cellulaires ou sous la forme de métabolites. Le ou le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.
Selon un mode de réalisation particulier, les microorganismes sont mis en oeuvre sous forme inactivée voire morte, notamment dans les compositions topiques.

Par prébiotique, on entend un composant alimentaire non-digestible capable de stimuler de façon sélective la croissance et/ou l'activité des bactéries probiotiques habituellement présentes dans le côlon pour un effet bénéfique sur l'organisme.

D'une façon générale, les oligosaccharides sont les principales sources de prébiotiques.
A ce titre, tous les FOS (Fructo Oligo Saccharides) sont des prébiotiques qui stimulent la croissance des Bifidobactéries. L'inuline et ses dérivés, trouvés dans la chicorée (*Ciichorium intybus*) et l'artichaut de Jérusalem (*Helianthus tuberosus*) et dans des Composées ou dans des Campanulacées sont également des promoteurs de Bifidobactéries. Les isomaltooligosaccharides tels que l'isomaltose en premier lieu mais également le panose, l'isomaltotréalose, le kojibiose... sont tous d'excellents stimulants de la croissance de Bifidobactéries et des lactobacilles.

L'utilisation selon l'invention se révèle particulièrement avantageuse lorsque le Lipide A de *Vitreoscilla filiformis* est administré en association avec des agents de peeling ou après application des agents de peeling pour stimuler les défenses endogènes de la peau par les keratinocytes.

Les peelings sont un moyen bien connu pour améliorer l'aspect et/ou la texture de la peau et/ou du cuir chevelu, notamment améliorer l'éclat et l'homogénéité du teint et/ou diminuer les irrégularités visibles et/ou tactiles de la peau, et en particulier pour améliorer l'aspect de surface de la peau, pour atténuer les lentigo actiniques, les marques d'acné ou de varicelle, ainsi que pour prévenir, atténuer ou lutter contres les signes du vieillissement cutané, et notamment pour lisser les irrégularités de la texture de la peau, telles les rides et les ridules.

Ils ont pour effet d'enlever une partie superficielle de la peau à traiter (épiderme et éventuellement couche superficielle du derme), par des méthodes chimiques.

Une conséquence de l'effet des agents de peeling ou desquamants est également l'élimination d'une partie de l'écoflore naturelle de la peau. L'utilisation concomitante de la fraction LPS selon l'invention permet de prévenir le développement d'une flore indésirable à la place de l'écoflore de la peau.

Par agent de peeling ou agent desquamant, on entend tout composé capable d'agir :
- soit directement sur la desquamation en favorisant l'exfoliation, tels que : les acides monocarboxyliques saturés et insaturés, les acides dicarboxyliques saturés et insaturés, les acides tricarboxyliques saturés et insaturés ; les alpha hydroxyacides et beta hydroxyacides des acides monocarboxyliques ; les alpha hydroxyacides et beta hydroxyacides des acides dicarboxyliques ; des alpha hydroxyacides et beta hydroxyacides des acides tricarboxiliques ; des kétoacides, des alpha kétoacides, des beta ketoacides d'acides polycarboxyliques, d'acides polyhydroxy monocarboxyliques, d'acides polyhydroxy bicarboxyliques, d'acides polyhydroxy tricarboxyliques ; et l'acide (3-hydroxy-2-pentylcyclopentyl)acétique.
Comme α-hydroxyacides préférés, on peut citer : l'acide glycolique, l'acide citrique, l'acide lactique, l'acide tartrique, l'acide malique ou l'acide mandélique.
Les β-hydroxyacides préférés sont choisis parmi : l'acide salicylique et ses dérivés, en particulier, l'acide n-octanoyl 5-salicylique.
Comme autres agents exfoliants, on peut citer : les acides dioique, pyruvique, gluconique, glucuronique, oxalique, malonique, succinique, acétique, gentisique, cinnamique, azélaique ; le phénol, la résorcine ; l'urée et ses dérivés ; les oligofucoses comme dans le brevet EP 0 218 200 ; l'acide jasmonique et ses dérivés comme dans les demandes de brevet EP 1 333 022 et EP 1 333 021 ; l'acide trichloracétique ; les rétinoides tels que le rétinol, l'acide rétinoique ; l'adapalène ; l'extrait de Saphora japonica ; le resvératrol ; ainsi que leurs sels et dérivés, tels que les formes cis ou trans, les mélanges racémiques, les formes dextrogyres ou levogyres des agents pré-cités.
- soit sur les enzymes impliquées dans la desquamation ou la dégradation des cornéodesmosomes, telles que les glycosidases, la stratum corneum chymotryptic enzyme (SCCE) voire d'autres protéases (trypsine, chymotrypsine-like). On peut citer notamment les agents chélatants des sels minéraux tels l'EDTA ; l'acide N-acyl-N,N',N'éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP 0 852 949, ainsi que le méthyl glycine diacétate de sodium commercialisé par BASF sous la dénomination commerciale TRILON M^{®}) ; le miel ; les dérivés de sucre tels que l'O-octanoyl-6-D-maltose, le O-linoleyl-6-D-glucose et la N-acétyl glucosamine.
Comme autres agents desquamants utilisables dans la composition selon l'invention, on peut citer, les extraits de laminaire tels que le laminaria saccharina et le laminaria ochrolenca, le trilactate de glycérol, les dérivés siliciés de salicylate comme dans le brevet EP 0 796 861, les sels d'acide 5-acyl salicylique, des actifs ayant des effets sur la transglutaminase comme dans le brevet EP 0 899 330, et un extrait de fleur de ficus opuntia indica comme Exfolactive^{®} de Silab.

L'homme du métier saura définir la quantité nécessaire d'agent desquamant présent dans l'association selon l'invention pour obtenir l'effet recherché sur la peau.
A titre d'exemple, l'agent desquamant pourra être en une teneur allant de 0,001 à 95% en poids par rapport au poids total de la composition, en particulier de 0, 01 à 30% en poids par rapport au poids total de la composition, préférentiellement de 0,01 à 10% en poids par rapport au poids total de la composition.

Ce Lipide A de *Vitreoscilla filiformis* trouve également des applications avantageuses dans l'ingénierie des modèles de peau.

Ainsi, l'invention se rapporte encore à l'utilisation du Lipide A de *Vitreoscidda filiformis* pour prévenir la prolifération microbienne dans les cultures cellulaires ou organotypiques. Cette utilisation peut venir en substitution ou en association avec des antibiotiques naturels ou synthétiques.
Une telle utilisation permet la préparation septique de modèles d'épidermes et/ou de peaux ou encore la préparation septique des explants de peau ou des greffes de cheveux.

Les modèles de peau peuvent être des épidermes générés à partir de cellules épithéliales, kératinocytes, progéniteurs, cellules souches.
Il pourra également et non exclusivement s'agir d'épidermes reconstruits sur membrane synthétique, sur bio-support collagénique, sur derme natif acellulaire dé-épidermisé (modèle DED), de dermes vivants comprenant des fibroblastes inclus dans un gel de collagène (modèle lattice) ou inclus dans une éponge de collagène.

Les modèles de peau reconstruite peuvent en outre comprendre des cellules non kératinocytaires telles que les cellules de Langerhans et/ou les mélanocytes, intégrées dans l'épiderme et/ou des cellules non fibroblastiques telles que les cellules endothéliales et/ou les lymphocytes, intégrées dans le derme.

Les modèles de peau comprennent également être des explants maintenus en survie *ex vivo.*

A titre d'exemple, on peut citer le modèle d'épiderme reconstruit EpiSkin®, obtenu par culture de kératinocytes humains adultes sur un support collagénique dans des conditions permettant leur différenciation terminale et la reconstruction d'un épiderme avec une couche cornée fonctionnelle.

La présente invention pourra s'appliquer aux différents modèles de peau disponibles commercialement : EpiSkin®, ainsi que notamment et non exclusivement SkinEthic®, Matek®, Natskin®.
Différents modèles d'épiderme reconstruit et/ou de peau reconstruite (modèles expérimentaux de recherche et/ou modèles disponibles commercialement) sont notamment décrits dans les références suivantes : Asselineau D, Bernard B, Bailly C, Darmon M (1985). Epidermal morphogenesis and induction of the 67 kD keratin polypeptide by culture of human keratinocytes at the liquid-air interface. Exp Cell Res. 159: 536-539. ;Bernerd F, Vioux C, Lejeune F, Asselineau D (2003). The sun protection factor (SPF) inadequately defines broad spectrum photoprotection: demonstration using skin reconstructed in vitro exposed to UVA, UVB or UV-solar simulated radiation. Eur J Dermatol. 13: 242-249; Lenoir MC, Bernard BA, Pautrat G, Darmon M, Shroot B (1988). Outer root sheath cells of human hair follicle are able to regenerate a fully differentiated epidermis in vitro. Dev Biol. 130: 610-620; Prunieras M, Regnier M, Woodley D (1983). Methods for cultivation of keratinocytes with an air-liquid interface. J Invest Dermatol. 81 (1 suppl): 28s-33s; Regnier M, Staquet MJ, Schmitt D, Schmidt R (1997). Integration of Langerhans cells into a pigmented reconstructed human epidermis. J Invest Dermatol. 109: 510-512; Regnier M, Desbas C, Bailly C, Darmon M (1988). Differentiation of normal and tumoral human keratinocytes cultured on dermis: reconstruction of either normal or tumoral architecture. In Vitro Cell Dev Biol. 24: 625-632; Regnier M, Schweizer J, Michel S, Bailly C, Prunieras M (1986). Expression of high molecular weight (67K) keratin in human keratinocytes cultured on dead de-epidermized dermis. Exp Cell Res. 165: 63-72; Tinois E, Tiollier J, Gaucherand M, Dumas H, Tardy M, Thivolet J (1991). In vitro and post-transplantation differentiation of human keratinocytes grown on the human type IV collagen film of a bilayered dermal substitute. Exp Cell Res. 193: 310-319.

Le procédé selon l'invention peut également être mis en oeuvre sur d'autres modèles d'épithélium. Il pourra notamment et non exclusivement s'agir de modèles d'épithélium oral, gingival, oesophagien, vaginal, cornéen (oculaire), alvéolaire (pulmonaire), tels que les modèles commerciaux SkinEthic®.

Différents modèles d'épithélium sont notamment décrits dans les références suivantes :
- *Oral et gingival :* Jayatilake JAMS, Samaranayake YH, Samaranayake LP (2005). An ultrastructural and a cytochemical study of candidal invasion of reconstituted human oral epithelium. J Orad Pathol Med. 34: 240-246; Vande Vannet B, Hanssens J-L (2004). Characterization of human oral and gingival mucosal models: a histological characterization and applications in toxicity testing. 3rd International SkinEthic Workshop, Nice, France.
- *Oesophagien :* Bernhardt J, Bernhardt H, Knoke M, Ludwig K (2004). Influence of voriconazole and fluconazole on reconstituted multilayered oesophageal epithelium infected by Candida albicans. Mycoses. 47, 7, p 330.
- *Vaginal :* Schaller M, Korting HC, Borelli C, Hamm G, Hube B (2005). Candida albicans-secreted aspartic proteinases (Sap) modify the epithelial cytokine response in an in vitro model of vaginal candidiasis. Infect Immun. 73: 2758-2765.
- *Cornéen :* Van Goethem F, Adriaens E, Alépée N, Straube F, De Wever B, Cappadoro M, Catoire S, Hansen E, Wolf A, Vanparys P (2006). Prevalidation of a new in vitro reconstituted human corneal model to assess the eye irritating potential of chemicals. Toxicology In Vitro. 20: 1-17.
- *Alvéodaire :* Roggen E, Nilsson N, Dam L, Mikkelsen B, Rasmussen C, Cappadoro M, Rosdy M (2001). The use of airlifted reconstituted human lung epithelial tissues to predict in vitro the allergenic risk of topically applied enzyme proteins. Congrès de la Société de pharmaco-toxicologie cellulaire (SPTC), Toulouse, France.

De préférence, le Lipide A de *Vitreoscilla filiformis* sera formulée dans une composition cosmétique ou pharmaceutique destinée à être appliquée topiquement sur la peau, le cuir chevelu ou les muqueuses.
Les compositions comprennent entre 0,001 ng/ml et 100 mg/ml et préférentiellement entre 1 ng/ml et 100 µg/ml de Lipide A de *Vitreoscilla filiformis.*

Les compositions utilisées selon l'invention peuvent se présenter sous toutes les formes adaptées aux applications envisagées, notamment par voie topique, dans les domaines cosmétiques et dermatologiques.

La composition selon l'invention peut ainsi être appliquée topiquement sur toute zone cutanée du corps, de la peau, du cuir chevelu ou des muqueuses et se présenter sous toute forme galénique adaptée par l'homme du métier. Elle peut se présenter notamment sous forme d'une solution ou d'une suspension aqueuse ou huileuse, d'une émulsion huile-dans-eau ou eau-dans-huile ou multiple, d'une émulsion siliconée, d'une microémulsion ou nanoémulsion, d'un gel aqueux ou huileux ou d'un produit anhydre liquide, pâteux ou solide.
Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydre, ou encore de microcapsules ou microparticules, ou de dispersions vésiculaires de type ionique et/ou non ionique. Elle peut également être présentée sous forme de patch ou de système à libération contrôlée. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des masques à laisser poser sur la peau ou les cheveux, des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes dépilatoires, des compositions pour traiter certaines maladies de la peau comme l'acné, l'eczéma, le psoriasis.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 à 80 % en poids, et de préférence de 5 à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 9 % du poids total de la composition.
De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones®, les sels métalliques d'acides gras comme les stéarates d'aluminium et la silice hydrophobe, éthylcellulose, polyéthylène.

Lorsque le Lipide A est contenu dans une fraction LPS spécifique (enrichie en Lipide A), la quantité de fraction de LPS de *Vitreaoscilla filiformis* présente dans les compositions selon l'invention sera adaptée par l'homme du métier pour obtenir l'activité régulatrice. A titre indicatif, la quantité de fraction LPS spécifique de *Vitreaoscilla filiformis* dans les compositions sera comprise entre 0,0001 et 10% en poids par rapport au poids total de la composition, de préférence de 0,001 à 2%; en particulier elle sera d'au moins 0,01 %.

Avantageusement, la composition selon l'invention comprendra des nanosphères ou des microsphères, telles que décrits par exemple dans les demandes de brevet EP 0 447 318, EP 0 557 489, EP 1 151741, EP 1 201 219 ou WO 97/12602.

### Exemple 1 - Préparation du Lipide A

### a) Synthèse de A

Une solution contenant le glucosamine commercial **A'** (1.4 mmol) et le 3-dodécanoyloxy décanoyle (1.54 mmol) dans CH₂Cl₂ (15 ml) est traitée avec EDC.MeI (2.10 mmol) et agitée à température ambiante toute la nuit. Le mélange réactionnel est ensuite concentré et le résidu est purifié par chromatographie flash sur gel de silice.

Le composé précédent (15,2 mmol) est mis en solution avec le 3-hydroxy décanoyle (16.7 mmol) et la 4-pyrrolidinopyridine (1.7 mmol) dans CH₂Cl₂ (95 mL). EDC.MeI (16.7 mmol) est ajouté et le milieu est agité à température ambiante toute la nuit. Le mélange réactionnel est ensuite concentré et le résidu est purifié par chromatographie flash sur gel de silice.

L'acétonide obtenu ci-dessus est dissout dans un mélange acide acétique / eau (4 / 1) et chauffé à 60°C pendant 1 h. Le milieu est ensuite concentré et purifié par chromatographie flash sur gel de silice pour obtenir l'intermédiaire **A.**

### b) Synthèse de B

**B'** est obtenu à partir du produit commercial **B"** en appliquant le même protocole utilisé pour la synthèse de **A** (voir ci-dessus).

TCBOC-Cl (13.2 mmol) dans CH₂Cl₂ (25 mL) est ajouté goutte à goutte pendant 15 min, à une solution à 0°C contenant **B'** (12 mmol) et pyridine (25 mmol) dans CH₂Cl₂ (125 ml). Le mélange est ensuite ramené doucement à température ambiante pendant 3,5 h. On ajoute successivement la 4-pyrrolidinopyridine (6.0 mmol), la N,N-diisopropyléthylamine (60 mmol), et le diphényl chlorophosphonate (18 mmol). Le mélange est agité à température ambiante pendant 5h. La réaction est alors diluée avec CH₂Cl₂, lavée avec une solution aqueuse de HCl (7.5 %) froide, puis avec une solution aqueuse saturée en NaHCO₃, puis séchée et concentrée. Le résidu est purifié par chromatographie flash sur gel de silice.

ZnCl₂ (1.0 M dans éther, 2.41 mmol) est ajouté à 0 °C à une solution contenant le composé obtenu ci-dessus (4.84 mmol) et du dichlorométhyl méthyl éther (24.2 mmol) dans CHCl₃ (60 mL). Le mélange est amené doucement à température ambiante, puis agité à température ambiante toute la nuit. Le milieu réactionnel est ensuite dilué avec EtOAc, lavé avec une solution aqueuse saturée en NaHCO₃, puis séchée et concentrée. Le résidu est purifié par chromatographie flash sur gel de silice pour obtenir le composé **B.**

### c) Synthèse de C

Une solution contenant **B** (1.85 mmol) et **A** (1.54 mmol) dansle 1,2-dichloroéthane (18.5 mmol) est agitée avec du tamis moléculaire 4 A (1g) pendant 1h, puis traitée avec AgOTf (5.55 mmol) en une seule portion. Après avoir agité 4h à température ambiante dans le noir, un supplément d'AgOTf (1.85 mmol) est ajouté et la réaction est agitée toute la nuit. Le mélange crémeux est ensuite filtré sur célite et concentré. Une purification par chromatographie flash sur gel de silice permet d'obtenir le composé **C'.**

Une solution contenant **C'** (0.46 mmol) dans un mélange AcOH (4.5 mL) / THF (40 mL) est hydrogénée en présence de PtO₂ (0.45 g) à température ambiante sous une pression de 70 psig pendant 18 h. La solution est diluée avec CHCl₃/MeOH (2/1), puis soniquée brièvement. Le catalyseur est ensuite filtré et lavé avec le mélange CHCl₃/MeOH (2/1). Les filtrats sont regroupés et concentrés. Le résidu est purifié par chromatographie flash sur gel de silice.

Le produit précédent (12 mmol) dans CH₂Cl₂ (25 mL) est agité en présence de N,N-diisopropyléthylamine (60 mmol), et POCl₃ (18 mmol) à température ambiante pendant 5h. La réaction est ensuite concentrée. Le milieu réactionnel est ensuite dilué avec CH₂Cl₂, lavé avec une solution aqueuse saturée en NaHCO₃, puis séché et concentré. Le résidu est ensuite dissout dans l'acide acétique (100 mL) et chauffé à 60°C avec de la poussière de Zinc (0.9 mmol). La réaction est ensuite refroidie et filtrée sur célite, puis concentrée. Une purification par chromatographie flash sur gel de silice permet d'obtenir le composé **C.**

### Exemple 2 - Préparation d'un extrait de Vitreoscilla filiformis contenant le Lipide A

125 grammes de lyophylisat de *Vitreoscilla filiformis* sont obtenus comme suit :
- culture en mode continu (µ= 0,12 H-1) en fermenteur de 3 m³ efficace équipé d'un draft tube ;
- récolte en continu par centrifugation (10.000 G) en conditions stériles;
- lyophilisation de la biomasse ainsi obtenue.

Ces 125 grammes de biomasse lyophilisée sont ensuite extraits par le mélange suivant :
- 33,5 ml de NH4OH concentré ;
- 906 ml d'eau osmosée ;
- 1560 ml d'acide isobutyrique.

L'extraction s'effectue durant 10 à 30 minutes sous agitation à température ambiante.

Le produit obtenu est centrifugé à 8000 G / 30'/4°C pour éliminer les particules. Le surnageant de centrifugation est ensuite filtré sur filtre GFD puis GFF.
NB : Le culot est la biomasse de *Vitreoscilla filiformis* débarrassée à 95% de LPS (Fraction appelée par la suite LPS free, utilisée dans l'exemple 4).
L'ensemble est alors évaporé sous vide à 65°C/40 mBar.
On obtient un concentré de 600 ml qui est placé en étuve ventilée pour être concentré. On obtient alors 37 g d'une pâte qui, additionnée avec 50% d'eau, est lyophilisée.

### EXEMPLE 3 - mesure de l'induction de l'expression d'enzymes de défenses cutanées par le Lipide A de Vitreoscilla filiformis, comparaison avec le LPS d'E. coli

Au moyen d'une analyse génomique (puces Affymetrix U133 plus), deux extraits de lipopolysaccharides bactériens purifiés, l'un correspondant à l'extrait LPS spécifique riche en Lipide A selon l'invention, l'autre étant un extrait LPS d*'Echerichia coli* d'origine commerciale, ont été évalués pour leur capacité à induire spécifiquement l'expression de gènes particuliers dans les cellules cutanées codant pour ces protéines antibactériennes.

Les kératinocytes épidermiques humains normaux (NHEK) sont pré-cultivés en milieu de culture SFM (Invitrogen) avec de l'Epidermal Growth Factor à 0,25 ng/ml et extraits pituitaires à 25 µg/ml (EGF, EP, Invitrogen 3700015) et de la Gentamycine à 25 µg/ml (Sigma G1397) puis placés en milieu d'essai.
Ils sont ensuite introduits en milieu d'essai (même milieu que pour la culture sans EP ni EGF)
Les cellules sont ensuite traitées ou non (témoin) par les produits à l'essai et cultivés pendant 24 heures à 37°C et 5% de CO₂.
Les surnageant de culture ont ensuite été éliminés et les tapis cellulaires ont été rincés avec une solution de PBS puis placés dans des tubes stériles RNA-free en présence de Tri-Reagent (Sigma T9424) et immédiatement congelés à -80°C.

L'expression des précurseurs de synthèse des protéines d'intérêt a été évaluée par RT-Q-PCR sur les ARN messagers extraits des tapis cellulaires de chaque traitement :

### Reverse transcription :

- extraction des ARN totaux de chaque échantillon à l'aide de Tri-Reagent selon le protocole préconisé par le fournisseur ;
- élimination des traces d'ADN potentiellement contaminant par traitement avec le système DNA-free (Ambion ref. 1906) ;
- réalisation de la réaction de réverse-transcription de l'ARNm en présence de l'amorce oligo(dT) et de l'enzyme SuperscriptII (Invitrogen 18064071) pour les études Q/RT PCR (exemple 4) ou au moyen du Kit de reverse transcription One cycle d'Affymetrix pour les études transcriptionelles Affymetrix (exemple 3).

Au moyen d'une analyse biostatistique couplée à une analyse de data mining sur l'ensemble du génome humain, il a ainsi été observé que, contrairement au LPS d'*E*. *coli* connu pour avoir peu d'activité stimulatrice sur les kératinocytes cutanés, le Lipide A de *Vitreoscilla filiformis,* aux mêmes concentrations et dans les mêmes conditions expérimentales était capable de stimuler pleinement la mise en route préventive des systèmes de défense cutanés, notamment anti-bactériens.
Ainsi, ce constituant bactérien ou mimétique a pour propriété biologique d'induire les systèmes de défense cutanés, en particulier, la production de beta defensine et la cathelicidin mais aussi des inhibiteurs de protéases comme l'Elafine (SKALP) ; Elias et al Exp. Dermatol (14) : 719-726.

Ainsi, comme l'indique le tableau ci-dessous, on constate bien en réponse au Lipide A de V. *filiformis* l'induction de mRNA codant pour la defensine beta 2, la RNase7, la PS100A7 mais aussi d'autres anti-proteases qui contribuent à la défense de l'épiderme.

| **Références Genbank** | **Description** | **Fraction LPS spécifique de *Vitreoscilla filiformis* riche en Lipide A selon l'invention sur kératinocytes humains normaux** | **Fraction LPS d'*E. coli* sur kératinocytes humains normaux** |
|---|---|---|---|
| NM_002963 | **psoriasin 1** (S100 calcium binding protein A7) | 14.8 | 1.4 |
| AJ243672 | S 100 calcium binding protein A7-like 1 | 14.7 | 1.2 |
| NM_004942 | **defensin beta 2** | 8.6 | 1.1 |
| AJ131212 | ribonuclease, RNase A family, 7 | 2.6 | -1.0 |
| AI554300 | serine (or cysteine) proteinase inhibitor, clade B (ovalbumin), member 1 | 3.5 | 1.3 |
| NM_002638 | protease inhibitor 3, skin-derived (SKALP) /// protease inhibitor 3, skin-derived (SKALP) | 3.5 | 1.3 |
| NM_030666 | serine (or cysteine) proteinase inhibitor, clade B (ovalbumin), member 1 | 3.4 | 1.2 |
| L10343 | protease inhibitor 3, skin-derived (SKALP) | 2.7 | 1.2 |

### Exemple 4 - Comparaison de l'expression de défensine beta 2 par les kératinocytes par la fraction selon l'invention, une fraction de Vitreoscilla filiformis sans LPS et une fraction LPS d'E. coli

Les réactions de PCR (Polymerase chain reactions) ont été réalisées par PCR quantitative avec le système « Light Cycler » (Roche Molecular Systems Inc.) et selon les procédures recommandées par le fournisseur

Le mélange réactionnel (10 µl final) pour chaque échantillon est le suivant :
- 2,5 µl d'ADNc dilué 1/10eme ;
- Amorces des différents marqueurs utilisés ;
- Mélange réactionnel (Roche) contenant l'enzyme taq DNA polymerase, le marqueur SYBR Green I (fluorophore qui s'intercale dans l'ADN double brin au cours de l'étape d'élongation) et du MgCl₂.

### Analyse par Q-PCR

L'incorporation de fluorescence dans l'ADN amplifié est mesurée en continu au cours des cycles de PCR. Ce système permet d'obtenir des courbes de mesure de la fluorescence en fonction des cycles de PCR et d'évaluer ainsi une valeur d'expression relative pour chaque marqueur.
Le nombre des cycles est déterminé à partir de points de « sorties » des courbes de fluorescence. Pour un même marqueur analysé, plus un échantillon sort tard (nombre de cycles élevé), plus le nombre initial de copies d'ARNm est faible.
La valeur de RE (relative expression) est exprimée en unités arbitraires selon la formule suivantes: 1/2^{nombre de cycles} x 10⁶

Le protocole de préparation des ARN de l'exemple 3 a été mis en oeuvre avec deux autres produits :
A- une fraction de *Vitreoscilla filiformis* sans LPS (fraction dite LPS free, voir exemple 1) telle que notée dans la préparation du LPS de *V. filiformis.* Il s'agit de la biomasse totale (protéines...) débarrassée à 95% de ses LPS
B- une fraction LPS d'*Escherichia coli* commerciale.

| **traitement** | **Concentration** | **Expression de defensin beta 2 % témoin** |
|---|---|---|
| Témoin | - | 100 |
| Fraction selon l'invention (préparée selon exemple 2) | 10 µg/ml | 221 |
| Fraction A | 0,1% | 155 |
| Fraction B | 10 µg/ml | 191 |

### Exemple 5 - Compositions

### A. Voie orale

### Exemple 1 : sachet poudre unidose

| **Principe actif** | **mg/sachet** |
|---|---|
| Extrait de Lipide A de *Vitreoscilla filiformis* (exemple 2) | 1 |
| Citrate de magnésium en mg de magnesium | 300 |
| inuline | 50 |
| Huile de bourrache | 100 |
| Huile de noix | 100 |
| **Excipient** | |
| Maltodextrine | Qsp |
| Benzoate de sodium | Qsp |

On peut prendre un à trois sachets par jour.

### Exemple 2 : capsule

| | **mg/capsule** |
|---|---|
| *Lactobacillus paracasei (CNCM I-2116)* | 50,00 |
| Extrait de Lipide A de *Vitreoscilla filiformis* (exemple 2) | 5,00 |
| Huile de pépins de cassis | 100,00 |
| Huile de poisson | 100,00 |
| Stéarate de magnésium | 0,02 |
| Arôme naturel | Qs |
| Excipient | Qsp |

On peut prendre une à trois de ces capsules par jour.

### B. Administration topique sur la peau

### Exemple 3 : Spray déodorant (flacon-pompe)

| | |
|---|---|
| Extrait de Lipide A de *Vitreoscilla filiformis* (exemple 2) | 0,30 g |
| Parfum, colorants | Qs |
| Alcool éthylique 95 % vol. dénaturé | 100 g |

### Exemple 4 : Roll-on hydroalcoolique transparent

| | |
|---|---|
| Alcool cétylique oxyéthyléné (20 OE) et oxypropyléné (5 OP) | 2,00 g |
| Hydroxyéthylcellulose | 0,75 g |
| Extrait de Lipide A de *Vitreoscilla filiformis* (exemple 2) | 0,0250 g |
| Alcool éthylique 95 % vol. dénaturé | 45,00 g |
| Parfum | qs |
| Eau déminéralisée qsp | 100 g |

### Exemple 5 : Gel masque antichute pour l'entretien du scalp

| (% en poids) | |
|---|---|
| *Lactobacillus johnsonii (CNCM 1-1225)* | 0,05 |
| Hydroxypropylcellulose (Klucel H vendu par la société Hercules) | 1,00 |
| Extrait de Lipide A de *Vitreoscilla filiformis* (exemple 2) | 5,00 |
| Gluconate de magnésium | 3,00 |
| Linoléate de calcium | 2,00 |
| Huile de pépins de cassis | 5,00 |
| Huile d'Onagre | 2,00 |
| Huile de bourrache | 5,00 |
| Antioxydant | 0,05 |
| Vitamine C | 2,50 |
| Antioxydant | 0,05 |
| Isopropanol | 40,00 |
| Conservateur | 0,30 |
| Eau qsp | 100,00 |

### Exemple 6 : Crème pour les pieds

| | |
|---|---|
| Mélange mono/distéarate de glycéryle / stéarate de polyethylène glycol (100 OE) | 8,00 g |
| Alcool cétylique | 2.50 g |
| Mélange cire microcristalline / cire d'abeille / polyéthylène | 1.00 g |
| Cire liquide de jojoba | 6.00 g |
| Myristate de myristyle oxyéthyléné (3 OE) | 2.00 g |
| Extrait de Lipide A de *Vitreoscilla filiformis* (exemple 2) | 0,4 g |
| Parfum, conservateurs | Qs |
| Eau déminéralisée qsp | 100 g |

### Exemple 7 : Lotion antipelliculaire pour le cuir chevelu

| | |
|---|---|
| Alcool éthylique 95% vol. dénaturé | 55.00 g |
| Extrait de Lipide A de *Vitreoscilla filiformis* (exemple 2) | 0,10 g |
| Propylène glycol | 10.00 g |
| Parfum, colorant | Qs |
| Eau déminéralisée qsp | 100 g |

## Revendications

1. Lipide A de *Vitreoscilla filiformis* obtenu par synthèse chimique ou extrait de *Vitreoscilla filiformis* **caractérisé en ce qu'**il est constitué d'un composé choisi parmi :
(a) les composés de formule (I) : 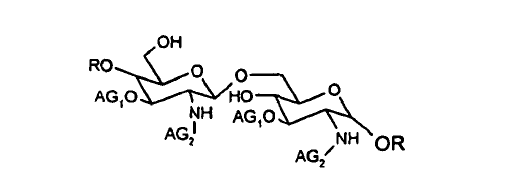 dans laquelle :
AG₁ désigne un groupe 3-hydroxy décanoyle
AG₂ désigne un groupe 3-dodécanoyloxy décanoyle,
où R désigne un atome d'hydrogène ou un groupe PO(OR')₂, R' désignant un atome d'hydrogène, un groupe alkyle linéaire ou ramifié, saturé ou insaturé, en C₁-C₆, ou un groupe phényle ou benzyle, et
(b) dans le cas où R désigne un groupe PO(OH)₂ dans la formule (I) ci-dessus, d'un sel inorganique du composé de formule (I), ou d'un sel d'amine primaire, secondaire ou tertiaire du composé de formule (I), ou d'un sel de phosphoéthanolamine du composé de formule (I), pour son utilisation pour induire l'expression de peptides anti-microbiens par la peau, les muqueuses, semi-muqueuses et le cuir chevelu pour inhiber la prolifération d'une flore microbienne.

2. Lipide A pour son utilisation selon la revendication 1, pour prévenir et/ou diminuer et/ou à inhiber la prolifération d'une flore microbienne cutanée indésirable.

3. Lipide A pour son utilisation selon la revendication 2, pour traiter des désordres cutanés liés au développement d'un microorganisme choisi parmi *Staphylococcus aureus, Streptococcus pyogenes, Pseudomonas aeruginosa, Escherichia coli, Clostridium pefringens, Clostridium difficile, Gardnerella vaginalis, Propionibacterium acnes, Klebsiella species, Streptopyogenes, Candida albicans, Malassezia furfur, Trichophyton mentagrophytes, Trichophyton interdigitale, Trichophyton rubrum, Trichophyton yaoundei, Tinea capitis. Tinea corporis* ou des fungi tels que des *Aspergillus sp.*

4. Lipide A pour son utilisation selon la revendication 3, **caractérisé en ce que** lesdits désordres cutanés sont choisis parmi les complications infectieuses des désordres dermatologiques ; l'acné ; des complications infectieuses pendant la cicatrisation ; des dermatophytoses ; des candidoses ; des vaginoses ; des onychomycoses ; des teignes du cuir chevelu, du corps ; des désordres cutanés liés à des thérapeutiques avec des antibiotiques ou des antimycosiques ou entraînés par des dysrégulations hormonales ; des polydermites ; des érysipèles ; des dermites séborrhéiques.

5. Lipide A pour son utilisation selon la revendication 4, pour prévenir et/ou traiter les infections ou surinfections microbiennes de la peau.

6. Lipide A pour son utilisation selon la revendication 5, pour prévenir et/ou traiter l'infection ou la surinfection des plaies ou lésions cutanées.

7. Lipide A pour son utilisation selon l'une quelconque des revendications 5 à 6, **caractérisé en ce que** les surinfections sont choisies parmi les dermatites atopiques surinfectées, l'eczéma impétiginisé, l'acné inflammatoire surinfecté, l'herpes surinfecté.

8. Lipide A pour son utilisation selon la revendication 7, pour traiter des surinfections pendant la cicatrisation choisies parmi les ulcères, les plaies, les brûlures.

9. Lipide A pour son utilisation selon la revendication 3, pour traiter des dermatophytoses choisies parmi la teigne du cuir chevelu, la teigne du corps, le pied d'athlète, l'eczéma marginé de Hebra, l'herpès circiné.

10. Lipide A pour son utilisation selon la revendication 3, pour traiter des candidoses choisies parmi les candidoses des muqueuses, les candidoses vaginales, les candidoses interdigitales, les candidoses liées à des professions à risques ou au diabète.

11. Lipide A pour son utilisation selon la revendication 3, pour traiter des polydermites choisies parmi l'impétigo, les folliculites superficielles.

12. Lipide A pour son utilisation selon l'une quelconque des revendications 1 à 2, pour maintenir et/ou restaurer une écoflore cutanée normale.

13. Lipide A pour son utilisation selon l'une quelconque des revendications 1 à 2, pour prévenir et/ou traiter les états pelliculaires du cuir chevelu.

14. Lipide A pour son utilisation selon l'une quelconque des revendications 1 à 2, pour prévenir et/ou traiter les états séborrhéiques de la peau et du cuir chevelu.

15. Lipide A pour son utilisation selon l'une quelconque des revendications 1 à 2, **caractérisé en ce qu'**il est présent dans une composition destinée à l'hygiène buccale.

16. Lipide A pour son utilisation selon l'une quelconque des revendications 1 à 2, pour prévenir et/ou limiter les mauvaises odeurs corporelles.

17. Lipide A pour son utilisation selon l'une quelconque des revendications précédentes, **caractérisé en ce qu'**il est appliqué topiquement sur la peau avec ou sans poil, le cuir chevelu, les muqueuses et/ou semi-muqueuses.

18. Lipide A pour son utilisation selon l'une quelconque des revendications précédentes, **caractérisé en ce qu'**il est présent dans une composition appliquée à un mammifère.

19. Lipide A pour son utilisation selon la revendication 18, pour un traitement vétérinaire.

20. Lipide A pour son utilisation selon la revendication 19, pour un usage vétérinaire pour le traitement et/ou la prévention des désordres liés à des infections staphylococciques, streptococciques et mycosiques.

21. Lipide A pour son utilisation selon l'une quelconque des revendications précédentes, **caractérisé en ce que** ladite fraction est utilisée en association avec un probiotique et/ou un prébiotique.

22. Lipide pour son utilisationA selon la revendication 21, **caractérisé en ce que** ladite fraction est utilisée en association avec un probiotique et un prébiotique.

23. Lipide A pour son utilisation selon la revendication 21 ou 22, **caractérisé en ce que** les probiotiques sont choisis parmi les ascomycètes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus. Lactococcus, Staphylococcus, Peptostrepococcus, Bacillus, Pediococcus, Micrococcus, Leuconostoc, Weissella, Aerococcus, Oenococcus, Lactobacillus.*

24. Lipide A pour son utilisation selon la revendication 23, **caractérisé en ce que** les probiotiques sont choisis parmi *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges.

25. Lipide A pour son utilisation selon la revendication 23 ou 24 **caractérisé en ce que** les probiotiques sont présents à une concentration allant de 10³ à 10¹² ufc, en particulier de 10⁵ à 10¹⁰ ufc et plus particulièrement de 10⁷ à 10⁹ ufc de microorganismes par gramme de support.

26. Lipide A pour son utilisation selon la revendication 22, **caractérisé en ce que** les prébiotiques sont choisis parmi les fructo oligo saccharides, l'inuline et les isomaltooligosaccharides.

27. Lipide A pour son utilisation selon la revendication 1, **caractérisé en ce qu'**il est utilisé simultanément ou après l'utilisation d'agent de peeling.

28. Lipide A pour son utilisation selon la revendication 27, **caractérisé en ce que** les agents de peeling sont choisis parmi les acides monocarboxyliques saturés et insaturés, les acides dicarboxyliques saturés et insaturés, les acides tricarboxyliques saturés et insaturés ; les alpha hydroxyacides et beta hydroxyacides des acides monocarboxyliques ; les alpha hydroxyacides et beta hydroxyacides des acides dicarboxyliques ; des alpha hydroxyacides et beta hydroxyacides des acides tricarboxiliques ; des kétoacides, des alpha kétoacides, des beta ketoacides d'acides polycarboxyliques, d'acides polyhydroxy monocarboxyliques, d'acides polyhydroxy bicarboxyliques, d'acides polyhydroxy tricarboxyliques ; et l'acide (3-hydroxy-2-pentylcyclopentyl)acétique, les acides pyruvique, gluconique, glucuronique, oxalique, malonique, succinique, acétique, gentisique, cinnamique, azélaique ; le phénol, la résorcine ; l'urée et ses dérivés ; les oligofucoses ; l'acide jasmonique et ses dérivés ; l'acide trichloracétique ; les rétinoides tels que le rétinol, l'acide rétinoique ; l'adapalène ; l'extrait de Saphora japonica ; le resvératrol ; ainsi que leurs sels et dérivés, tels que les formes cis ou trans, les mélanges racémiques, les formes dextrogyres ou levogyres des agents pré-cités ; les glycosidases, la stratum corneum chymotryptic enzym (SCCE) ou d'autres protéases (trypsine, chymotrypsine-like) ; les agents chélatants des sels minéraux tels l'EDTA ; l'acide N-acyl-N,N',N'éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine, le méthyl glycine diacétate de sodium ; le miel ; les dérivés de sucre tels que l'O-octanoyl-6-D-maltose, le O-linoleyl-6-D-glucose et la N-acétyl glucosamine ; les extraits de laminaire tels que le laminaria saccharina et le laminaria ochrolenca, le trilactate de glycérol, les dérivés siliciés de salicylate, les sels d'acide 5-acyl salicylique, des actifs ayant des effets sur la transglutaminase, et un extrait de fleur de ficus opuntia indica.

29. Utilisation du Lipide A de *Vitreoscilla filiformis* constitué par (a) les composés de formule (1): 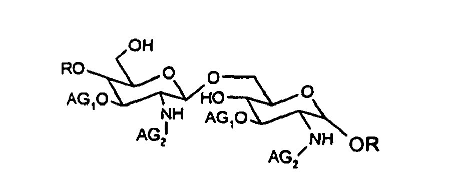 dans laquelle :
AG₁ désigne un groupe 3-hydroxy décanoyle
AG₂ désigne un groupe 3-dodécanoyloxy décanoyle,
où R désigne un atome d'hydrogène ou un groupe PO(OR')₂, R' désignant un atome d'hydrogène, un groupe alkyle linéaire ou ramifié, saturé ou insaturé, en C₁-C₆, ou un groupe phényle ou benzyle, et
(b) dans le cas où R désigne un groupe PO(OH)₂ dans la formule (I) ci-dessus, d'un sel inorganique du composé de formule (I), ou d'un sel d'amine primaire, secondaire ou tertiaire du composé de formule (I), ou d'un sel de phosphoéthanolamine du composé de formule (I), pour prévenir la prolifération microbienne dans les cultures cellulaires ou organotypiques.

30. Utilisation selon la revendication 29, pour la préparation septique de modèles d'épidermes et/ou de peaux.

31. Utilisation selon la revendication 30, pour la préparation septique des explants de peau ou des greffes de cheveux.

## Claims

1. Lipid A of *Vitreoscilla filiformis* obtained by chemical synthesis or extracted from vitreoscilla filiformis, **characterized in that** it consists of a compound chosen from:
a) the compounds of formula (I): 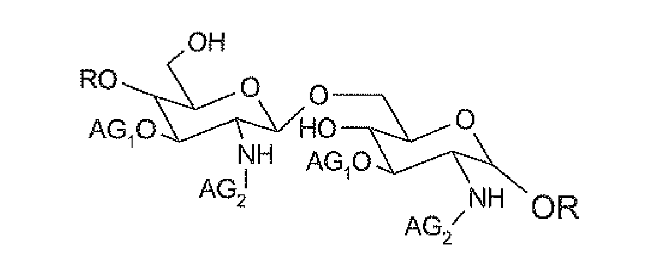 in which:
AG₁ denotes a 3-hydroxydecanoyl group,
AG₂ denotes a 3-dodecanoyloxydecanoyl group,
where R denotes a hydrogen atom or a group PO(OR')₂, R' denoting a hydrogen atom, a linear or branched, saturated or unsaturated C₁-C₆ alkyl group, or a phenyl or benzyl group, and
(b) in the case where R denotes a group PO(OH)₂ in formula (I) above, an inorganic salt of the compound of formula (I), or a primary, secondary or tertiary amine salt of the compound of formula (I), or a phospho-ethanolamine salt of the compound of the formula (I), for use in inducing the expression of antimicrobial peptides by the skin, the mucous membranes, the semi-mucous membranes and the scalp in order to inhibit the proliferation of microbial flora.

2. Lipid A for use according to Claim 1, in preventing and/or reducing and/or inhibiting the proliferation of an unwanted microbial flora of the skin.

3. Lipid A for use according to Claim 2, in the treatment of skin disorders linked to the development of a microorganism chosen from *Staphylococcus aureus, Streptococcus pyogenes, Pseudomonas aeruginosa, Escherichia coli, Clostridium pefringens, Clostridium difficile, Gardnerella vaginalis, Propionibacterium acnes, Klebsiella species, Streptopyogenes, Candida albicans, Malassezia furfur, Trichophyton mentagrophytes, Trichophyton interdigitale, Trichophyton rubrum, Trichophyton yaoundei, Tinea capitis, Tinea corporis,* or fungi such as *Aspergillus sp.*.

4. Lipid A for use according to Claim 3, **characterized in that** said disorders are chosen from infectious complications of dermatological disorders; acne; infectious complications during cicatrization; dermatophytoses; candidiases; vaginoses; onychomycoses; scalp ringworm, body ringworm; skin disorders linked to therapies with antibiotics or antimycotic agents or brought about by hormone disturbances; dermatitis; erysipelas; seborrheic dermatitis.

5. Lipid A for use according to Claim 4, in preventing and/or treating microbial infections or superinfections of the skin.

6. Lipid A for use according to Claim 5, in preventing and/or treating the infection or the superinfection of skin wounds or lesions.

7. Lipid A for use according to either of Claims 5 and 6, **characterized in that** the superinfections are chosen from superinfected atopic dermatitis, impetiginous eczema, superinfected inflammatory acne and superinfected herpes.

8. Lipid A for use according to Claim 7, in the treatment of superinfections during cicatrization and which are chosen from ulcers, wounds and burns.

9. Lipid A for use according to Claim 3, in the treatment of dermatophytoses chosen from scalp ringworm, body ringworm, athlete's foot, Hebra's eczema marginatum and herpes circinatus.

10. Lipid A for use according to Claim 3, in the treatment of candidiases chosen from mucosal candidiases, vaginal candidiases, interdigital candidiases, candidiases linked to professions at risk or to diabetes.

11. Lipid A for use according to Claim 3, in the treatment of dermatites chosen from impetigo and superficial folliculitis.

12. Lipid A for use according to either one of Claims 1 and 2, in maintaining and/or restoring a normal skin ecoflora.

13. Lipid A for use according to either one of Claims 1 and 2, in preventing and/or treating dandruff conditions of the scalp.

14. Lipid A for use according to either one of Claims 1 and 2, in preventing and/or treating seborrheic conditions of the skin and of the scalp.

15. Lipid A for use according to either one of Claims 1 and 2, **characterized in that** it is present in a composition intended for oral hygiene.

16. Lipid A for use according to either one of Claims 1 and 2, in preventing and/or limiting unpleasant body odours.

17. Lipid A for use according to any one of the preceding claims, **characterized in that** it is applied topically to the skin with or without body hair, the scalp, the mucous membranes and/or the semi-mucous membranes.

18. Lipid A for use according to any one of the preceding claims, **characterized in that** it is present in a composition applied to a mammal.

19. Lipid A for use according to Claim 18, in a veterinary treatment.

20. Lipid A for use according to Claim 19, for veterinary use in the treatment and/or prevention of disorders linked to staphylococcal, streptococcal or mycotic infections.

21. Lipid A for use according to any one of the preceding claims, **characterized in that** it is used in combination with a probiotic and/or a prebiotic.

22. Lipid A for use according to Claim 21, **characterized in that** it is used in combination with a probiotic and a prebiotic.

23. Lipid A for use according to Claim 21 or 22, **characterized in that** the probiotics are chosen from ascomycetes such as *Saccharomyces, Yarrowia, Kluyveromyces, Torulaspora, Schizosaccharomyces pombe, Debaromyces, Candida, Pichia, Aspergillus* and *Penicillium,* and bacteria of the genus *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus* or *Lactobacillus.*

24. Lipid A for use according to Claim 23, **characterized in that** the probiotics are chosen from *Lactobacillus johnsonii, Lactobacillus reuteri, Lactobacillus rhamnosus, Lactobacillus paracasei, Lactobacillus casei* or *Bifidobacterium bifidum, Bifidobacterium breve, Bifidobacterium longum, Bifidobacterium animalis, Bifidobacterium lactis, Bifidobacterium infantis, Bifidobacterium adolescentis* or *Bifidobacterium pseudocatenulatum,* and mixtures thereof.

25. Lipid A for use according to Claim 23 or 24, **characterized in that** the probiotics are present at a concentration ranging from 10³ to 10¹² cfu, in particular from 10⁵ to 10¹⁰ cfu, and more particularly from 10⁷ to 10⁹ cfu of microorganisms per gram of support.

26. Lipid A for use according to Claim 22, **characterized in that** the prebiotics are chosen from fructooligosaccharides, inulin and isomaltooligosaccharides.

27. Lipid A for use according to Claim 1, **characterized in that** it is used simultaneously with or after the use of a peeling agent.

28. Lipid A for use according to Claim 27, **characterized in that** the peeling agents are chosen from saturated and unsaturated monocarboxylic acids, saturated and unsaturated dicarboxylic acids, saturated and unsaturated tricarboxylic acids; alpha-hydroxy acids and beta-hydroxy acids of monocarboxylic acids; alpha-hydroxy acids and beta-hydroxy acids of dicarboxylic acids; alpha-hydroxy acids and beta-hydroxy acids of tricarboxylic acids; keto acids, alpha-keto acids, beta-keto acids of polycarboxylic acids, of polyhydroxymonocarboxylic acids, of polyhydroxydicarboxylic acids, of polyhydroxytricarboxylic acids; and (3-hydroxy-2-pentylcyclopentyl)acetic acid, pyruvic acid, gluconic acid, glucuronic acid, oxalic acid, malonic acid, succinic acid, acetic acid, gentisic acid, cinnamic acid, azelaic acid; phenol, resorcinol; urea and derivatives thereof; oligofucoses; jasmonic acid and derivatives thereof; trichloroacetic acid; retinoids such as retinol or retinoic acid; adapalene; extract of *Saphora japonica;* resveratrol; and also salts and derivatives thereof, such as the *cis* or *trans* forms, racemic mixtures, and the dextrorotatory or levorotatory forms of the abovementioned agents; glycosidases, stratum corneum chymotryptic enzyme (SCCE) or other proteases (trypsin, chymotrypsin-like); mineral salt chelating agents such as EDTA; N-acyl-N,N',N'-ethylenediaminetriacetic acid; aminosulphonic compounds, and in particular (N-2-hydroxyethylpiperazine-N-2-ethane)sulphonic acid (HEPES); derivatives of 2-oxothiazolidine-4-carboxylic acid (procysteine); derivatives of alpha-amino acids of glycine type, sodium methyl glycine diacetate; honey; sugar derivatives such as O-octanoyl-6-D-maltose, O-linoleyl-6-D-glucose and N-acetylglucosamine; extracts of laminaria such as *Laminaria saccharina* and *Laminaria ochrolenca,* glyceryl trilactate, siliceous salicylate derivatives, 5-acylsalicylic acid salts, active agents with effects on transglutaminase, and an extract of *Ficus opuntia indica* blossom.

29. Use of the lipid A of *Vitreoscilla filiformis* consisting of
a) the compounds of formula (I): 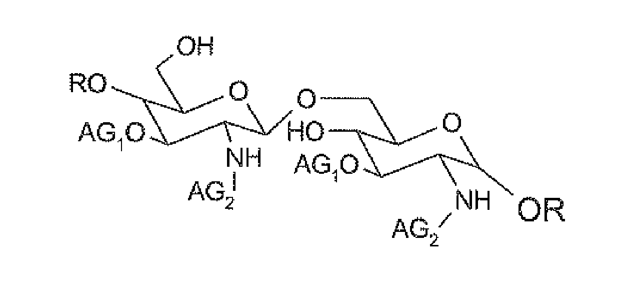 in which:
AG₁ denotes a 3-hydroxydecanoyl group,
AG₂ denotes a 3-dodecanoyloxydecanoyl group,
where R denotes a hydrogen atom or a group PO(OR')₂, R' denoting a hydrogen atom, a linear or branched, saturated or unsaturated C₁-C₆ alkyl group, or a phenyl or benzyl group, and
(b) in the case where R denotes a group PO(OH)₂ in formula (I) above, an inorganic salt of the compound of formula (I), or a primary, secondary or tertiary amine salt of the compound of formula (I), or a phospho-ethanolamine salt of the compound of the formula (I), for preventing microbial proliferation in cell or organotypic cultures.

30. Use according to Claim 29, for the septic preparation of epidermal and/or skin models.

31. Use according to Claim 30, for the septic preparation of skin explants or of hair grafts.

## Patentansprüche

1. Lipid A von *Vitreoscilla filiformis,* das durch chemische Synthese oder Extrakt aus *vitreoscilla filiformis* erhalten wird, **dadurch gekennzeichnet, dass** es aus einer Verbindung besteht, die ausgewählt wird aus:
(a) den Verbindungen der Formel (I): 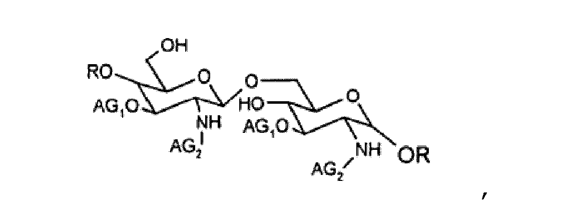 worin:
AG₁ eine 3-Hydroxydecanoyl-Gruppe bedeutet,
AG₂ eine 3-Dodecanoyloxydecanoyl-Gruppe bedeutet,
wobei R für ein Wasserstoffatom oder eine Gruppe PO(OR')₂ steht, worin R' ein Wasserstoffatom, eine gerade oder verzweigte, gesättigte oder ungesättigte C₁-C₆-Alkylgruppe oder eine Phenyl- oder Benzylgruppe bedeutet, und
(b) falls in der vorstehenden Formel (I) R für eine Gruppe PO(OH)₂ steht, einem anorganischen Salz der Verbindung der Formel (I) oder einem primären, sekundären oder tertiären Aminsalz der Verbindung der Formel (I) oder einem Phosphoethanolaminsalz der Verbindung der Formel (I), für die Verwendung zur Induktion der Expression antimikrobieller Peptide durch die Haut, die Schleimhäute, die Halbschleimhäute und die Kopfhaut, so dass die Proliferation einer mikrobiellen Flora gehemmt wird.

2. Lipid A für die Verwendung nach Anspruch 1 zur Vorbeugung und/oder Verringerung und/oder Hemmung der Proliferation einer unerwünschten mikrobiellen Flora der Haut.

3. Lipid A für die Verwendung nach Anspruch 2, zur Behandlung von Hautstörungen im Zusammenhang mit der Entwicklung eines Mikroorganismus, der aus *Staphylococcus aureus, Streptococcus pyogenes, Pseudomonas aeruginosa, Escherichia coli, Clostridium pefringens, Clostridium difficile, Gardnerella vaginalis, Propionibacterium acnes, Klebsiella species, Streptopyogenes, Candida albicans, Malassezia furfur, Trichophyton mentagrophytes, Trichophyton interdigitale, Trichophyton rubrum, Trichophyton yaoundei, Tinea capitis, Tinea corporis* oder Pilzen, wie *Aspergillus sp.,* ausgewählt wird.

4. Lipid A für die Verwendung nach Anspruch 3, **dadurch gekennzeichnet, dass** die Hautstörungen aus infektiösen Komplikationen dermatologischer Erkrankungen, Akne, infektiösen Komplikationen während der Narbenbildung, Dermatophytosen, Candidosen, Vaginosen, Onychomykosen, Tinea der Kopfhaut, des Körpers, Hautstörungen, die von Therapien mit Antibiotika oder Antimykotika herrühren oder durch hormonelle Dysregulationen hervorgerufen werden, Polydermatiden, Erysipel, seborrhoischen Dermatiden ausgewählt werden.

5. Lipid A für die Verwendung nach Anspruch 4 zur Vorbeugung und/oder Behandlung von mikrobiellen Infektionen oder Superinfektionen der Haut.

6. Lipid A für die Verwendung nach Anspruch 5 zur Vorbeugung und/oder Behandlung von Infektion oder Superinfektion von Hautwunden oder -läsionen.

7. Lipid A für die Verwendung nach einem der Ansprüche 5 bis 6, **dadurch gekennzeichnet, dass** die Superinfektionen aus superinfizierter atopischer Dermatitis, Eczema impetiginosa, superinfizierter entzündlicher Akne, superinfiziertem Herpes ausgewählt sind.

8. Lipid A für die Verwendung nach Anspruch 7 zur Behandlung von Superinfektionen während der Narbenbildung, ausgewählt aus Geschwüren, Wunden, Verbrennungen.

9. Lipid A für die Verwendung nach Anspruch 3 zur Behandlung von Dermatophytosen, die aus Tinea der Kopfhaut, Tinea des Körpers, Sportlerfuß, Eczema marginatum Hebra und Herpes circinatus ausgewählt sind.

10. Lipid A für die Verwendung nach Anspruch 3 zur Behandlung von Candidosen, die aus Candidosen der Schleimhäute, vaginalen Candidosen, interdigitalen Candidosen, Candidosen, die durch Risikoberufe oder Diabetes hervorgerufen werden, ausgewählt sind.

11. Lipid A für die Verwendung nach Anspruch 3 zur Behandlung von Polydermatiden, die aus Impetigo und oberflächlicher Follikulitis ausgewählt sind.

12. Lipid A für die Verwendung nach einem der Ansprüche 1 bis 2 zur Aufrechterhaltung und/oder Wiederherstellung einer normalen Ökoflora der Haut.

13. Lipid A für die Verwendung nach einem der Ansprüche 1 bis 2 zur Vorbeugung und/oder Behandlung von schuppiger Kopfhaut.

14. Lipid A für die Verwendung nach einem der Ansprüche 1 bis 2 zur Vorbeugung und/oder Behandlung von seborrhoischen Zuständen der Haut und der Kopfhaut.

15. Lipid A für die Verwendung nach einem der Ansprüche 1 bis 2, **dadurch gekennzeichnet, dass** es in einer Zusammensetzung für die Mundhygiene zugegen ist.

16. Lipid A für die Verwendung nach einem der Ansprüche 1 bis 2 zur Vorbeugung und/oder Eindämmung unangenehmer Körpergerüche.

17. Lipid A für die Verwendung nach einem der vorhergehenden Ansprüche, **dadurch gekennzeichnet, dass** es topisch auf die Haut mit oder ohne Körperbehaarung, die Kopfhaut, die Schleimhäute und/oder die Halbschleimhäute aufgetragen wird.

18. Lipid A für die Verwendung nach einem der vorhergehenden Ansprüche, **dadurch gekennzeichnet, dass** es in einer Zusammensetzung zugegen ist, die bei einem Säugetier aufgetragen wird.

19. Lipid A für die Verwendung nach Anspruch 18 für eine tierärztliche Behandlung.

20. Lipid A für die Verwendung nach Anspruch 19 für eine tierärztliche Verwendung zur Behandlung und/oder Vorbeugung von Erkrankungen im Zusammenhang mit Staphylokokken-, Streptokokken- oder mykotischen Infektionen.

21. Lipid A für die Verwendung nach einem der vorhergehenden Ansprüche, **dadurch gekennzeichnet, dass** es in Verbindung mit einem Probiotikum und/oder einem Präbiotikum verwendet wird.

22. Lipid A für die Verwendung nach Anspruch 21, **dadurch gekennzeichnet, dass** es in Verbindung mit einem Probiotikum und einem Präbiotikum verwendet wird.

23. Lipid A für die Verwendung nach Anspruch 21 oder 22, **dadurch gekennzeichnet, dass** die Probiotika aus Ascomyceten, wie *Saccharomyces, Yarrowia, Kluyveromyces, Torulaspora, Schizosaccharomyces pombe, Debaromyces, Candida, Pichia, Aspergillus* und *Penicillium,* Bakterien der Gattung *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus* ausgewählt sind.

24. Lipid A für die Verwendung nach Anspruch 23, **dadurch gekennzeichnet, dass** die Probiotika aus *Lactobacillus johnsonii, Lactobacillus reuteri, Lactobacillus rhamnosus, Lactobacillus paracasei, Lactobacillus casei* oder *Bifidobacterium bifidum, Bifidobacterium breve, Bifidobacterium longum, Bifidobacterium animalis, Bifidobacterium lactis, Bifidobacterium infantis, Bifidobacterium adolescentis* oder *Bifidobacterium pseudocatenulatum* und deren Gemischen ausgewählt ist.

25. Lipid A für die Verwendung nach Anspruch 23 oder 24, **dadurch gekennzeichnet, dass** die Probiotika in einer Konzentration von 10³ bis 10¹² cfu, insbesondere von 10⁵ bis 10¹⁰ cfu und ganz besonders 10⁷ bis 10⁹ cfu Mikroorganismen pro Gramm Träger vorliegen.

26. Lipid A für die Verwendung nach Anspruch 22, **dadurch gekennzeichnet, dass** die Präbiotika aus Fructooligosacchariden, Inulin und Isomaltooligosacchariden ausgewählt sind.

27. Lipid A für die Verwendung nach Anspruch 1, **dadurch gekennzeichnet, dass** es gleichzeitig mit oder nach der Verwendung eines Schälmittels verwendet wird.

28. Lipid A für die Verwendung nach Anspruch 27, **dadurch gekennzeichnet, dass** die Schälmittel aus gesättigten und ungesättigten Monocarbonsäuren, gesättigten und ungesättigten Dicarbonsäuren, gesättigten und ungesättigten Tricarbonsäuren, alpha-Hydroxysäuren und beta-Hydroxysäuren von Monocarbonsäuren, alpha-Hydroxysäuren und beta-Hydroxysäuren von Dicarbonsäuren, alpha-Hydroxysäuren und beta-Hydroxysäuren von Tricarbonsäuren, Ketosäuren, alpha-Ketosäuren, beta-Ketosäuren von Polycarbonsäuren, von Polyhydroxymonocarbonsäuren, von Polyhydroxydicarbonsäuren, von Polyhydroxytricarbonsäuren; und (3-Hydroxy-2-pentylcyclopentyl)essigsäure, Brenztraubensäure, Gluconsäure, Glucuronsäure, Oxalsäure, Malonsäure, Bernsteinsäure, Essigsäure, Gentisinsäure, Zimtsäure, Azelainsäure, Phenol, Resorcin, Harnstoff und seinen Derivaten, Oligofucosen, Jasmonsäure und deren Derivaten, Trichloressigsäure, Retinoiden, wie Retinol, Retinsäure, Adapalen, Extrakt von Saphora japonica, Resveratrol sowie deren Salzen und Derivaten, wie den cis- oder trans-Formen, racemischen Gemischen, rechtsdrehenden oder linksdrehenden Formen der vorstehend genannten Mittel; Glycosidasen, Stratum corneum chymotryptischem Enzym (SCCE) oder anderen Proteasen (Trypsin, Chymotrypsin-ähnlich), chelatisierenden Mitteln für Mineralsalze, wie EDTA, N-Acyl-N,N',N'-Ethylendiamintriessigsäure; Aminosulfonsäure-Verbindungen und insbesondere (N-2-Hydroxyethylpiperazin-N-2-ethan)sulfonsäure (HEPES), Derivaten von 2-Oxothiazolidin-4-carbonsäure (Procystein), Derivaten von alpha-Aminosäuren des Typs Glycin, Natriummethylglycindiacetat; Honig, Zuckerderivaten, wie O-Octanoyl-6-D-maltose, O-Linoleyl-6-D-glucose und N-Acetylglucosamin, Extrakten aus Laminaria, wie Laminaria saccharina und Laminaria ochrolenca, Glycerintrilactat, siliciumhaltige Salicylat-Derivate, Salze der 5-Acylsalicylsäure, Wirkstoffe mit Wirkungen auf die Transglutaminase und einem Extrakt aus der Blüte von Opuntia ficus-indica ausgewählt werden.

29. Verwendung von Lipid A von *Vitreoscilla filiformis*, bestehend aus (a) den Verbindungen der Formel (I): 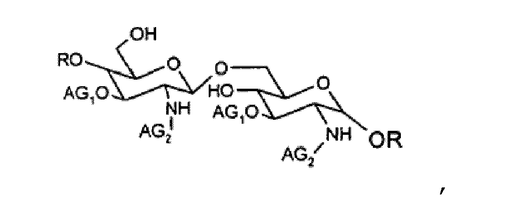 worin:
AG₁ eine 3-Hydroxydecanoyl-Gruppe bedeutet,
AG₂ eine 3-Dodecanoyloxydecanoyl-Gruppe bedeutet,
wobei R für ein Wasserstoffatom oder eine Gruppe PO(OR')₂ steht, worin R' ein Wasserstoffatom, eine gerade oder verzweigte, gesättigte oder ungesättigte C₁-C₆-Alkylgruppe oder eine Phenyl- oder Benzylgruppe bedeutet, und
(b) falls in der vorstehenden Formel (I) R für eine Gruppe PO(OH)₂ steht, einem anorganischen Salz der Verbindung der Formel (I) oder einem primären, sekundären oder tertiären Aminsalz der Verbindung der Formel (I) oder einem Phosphoethanolaminsalz der Verbindung der Formel (I), zur Vorbeugung der Proliferation von Mikroben in Zellkulturen oder organotypischen Kulturen.

30. Verwendung nach Anspruch 29 zur septischen Herstellung von Epidermis- und/oder Haut-Modellen.

31. Verwendung nach Anspruch 30, zur septischen Herstellung von Hautexplantaten oder Haartransplantaten.
